# EUROPEAN PATENT APPLICATION

(11) **EP 4 740 835 A1**
(43) Date of publication of application: **13.05.2026**
(21) Application number: 23944436.7
(22) Date of filing: 18.10.2023
(51) Int. Cl.: A61B 3/10, A61B 3/14

(54) **OPHTHALMOLOGIC IMAGING APPARATUS**

(30) Priority: 05.07.2023 JP 2023111073
(71) Applicant: NIDEK CO., LTD., Gamagori-shi, Aichi 443-0038 (JP)
(72) Inventor: TAKAYA, Ai, Gamagori-shi Aichi 443-0038 (JP); SATAKE, Norimasa, Gamagori-shi Aichi 443-0038 (JP)
(74) Representative: Hoefer & Partner Patentanwälte mbB
(86) International application number: PCT/JP2023/037711
(87) International publication number: WO 2025/009189

(57) **Abstract**

The present invention comprises: an imaging unit; a user interface; and a control means that causes a selection screen to display a plurality of inspection selection buttons corresponding to a preset inspection pattern, and receives a selection operation for any inspection selection button on the selection screen and, after receiving the selection operation, causes the display to transition to a second screen for displaying an observation image and adjusting the device state, and thus executes the inspection after passing through the display of the second screen, wherein the inspection selection buttons have identification information indicating the type of scan pattern, at least one of the inspection selection buttons has two or more types of identification information corresponding to two or more scan patterns, and when the inspection selection button on which the two or more types of identification information are arranged is selected and the inspection is started, the control means scans the eye to be examined with each scan pattern corresponding to the two or more types of identification information, and sequentially acquires OCT data of the eye to be examined corresponding to each scan pattern.

## Description

### TECHNICAL FIELD

The present disclosure relates to an ophthalmologic imaging apparatus.

### BACKGROUND ART

In the related art, ophthalmologic imaging apparatuses such as optical coherence tomography (OCT), fundus cameras, and SLO are widely used in medical institutions and examination facilities (hereinafter collectively referred to as "facilities and the like").

In OCT, an OCT optical system is controlled to scan an acquisition position of a subject eye with measurement light, thereby acquiring a tomographic image of the subject eye as an OCT image. In OCT, one of a variety of scan patterns, such as line scan, cross scan, multi-scan, radial scan, and raster scan, is selected for each examination. A graphical user interface (GUI) is used for an examiner to select the scan pattern.

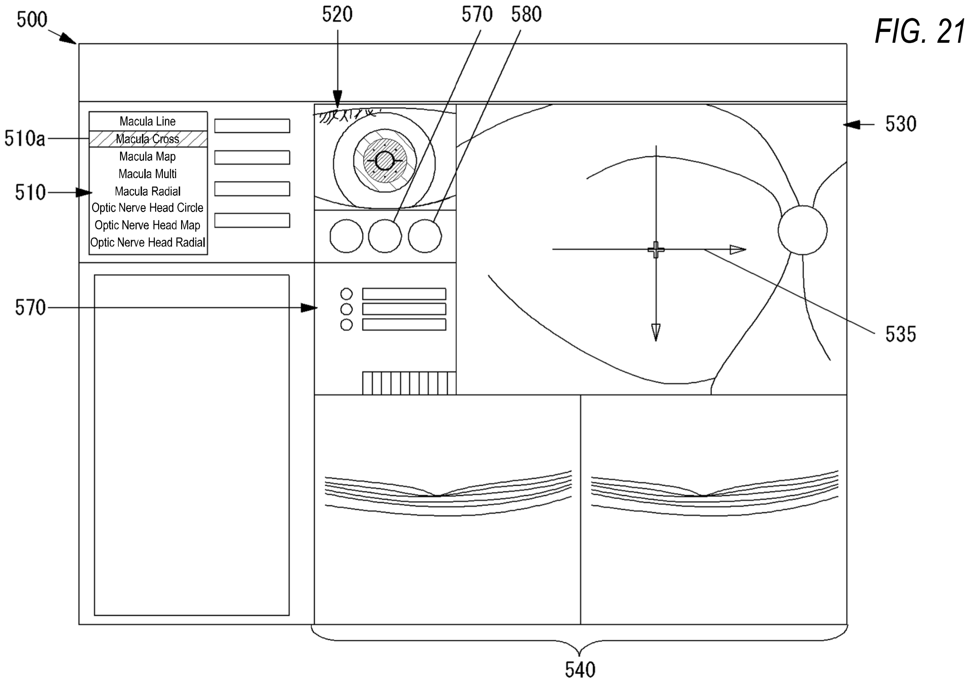

For example, in Patent Document 1, when selecting a scan pattern, a UI element on a screen 500 shown in FIG. 21 is operated. On the screen 500 shown in FIG. 21, a list 510, which is a UI element for selecting a scan pattern, is displayed simultaneously with an observation image (anterior ocular segment observation image 520), a fundus front image 530, and an OCT image 540. In the screen 500 shown in FIG. 21, the anterior ocular segment observation image 520 is used for manual or automatic alignment adjustment. On the screen 500, a UI element 550 for setting scan parameters, a UI element 560 for manual adjustment of the optical system, an optimize button 570 for automatic adjustment, a capture button 580, and the like are arranged. That is, UI elements for inputting various operations required from the selection of a scan pattern to the start of capture are arranged on the same screen.

In the list 510, a plurality of scan patterns are listed. By selecting a text 510a of the desired scan pattern on the list 510 based on the operation, one of the scan patterns is alternatively selected. A scan line 535 corresponding to the selected scan pattern is displayed on the fundus front image 530. The fundus front image 530 and the scan line 535 allow the scan pattern and a scan position to be confirmed. The OCT image 540 is acquired and displayed in real time at the scan position indicated by the scan line 535. In FIG. 21, two OCT images that form the scan pattern of the cross scan are displayed side by side. The scan line 535 can be moved on the fundus front image 530 in response to an operation, thereby changing the scan position.

### CITATION LIST

### PATENT LITERATURE

Patent Literature 1: JP2014-90748A

### SUMMARY OF INVENTION

In the screen configuration of the related art described above, it is difficult for an examiner who is unfamiliar with OCT examination to handle the screen because the configuration in one screen is complicated. In addition, among medical institutions and examination facilities (hereinafter, collectively referred to as "facilities and the like"), there are many facilities and the like in which examinations are performed on most subjects by using only a few examination patterns (combinations of one or more scan patterns). In such a facility, it is desirable to be able to efficiently perform examinations using a few predetermined examination patterns with a simpler procedure.

The present disclosure has been made based on at least one of the problems of the related art, and a technical object of the present disclosure is to provide an ophthalmologic imaging apparatus that easily improves examination efficiency.

An ophthalmologic imaging apparatus according to a first aspect of the present disclosure includes: an imaging unit including all or a part of an OCT optical system configured to scan tissue of a subject eye with a plurality of scan patterns; a user interface configured to receive an operation input by an examiner; and a controller configured to display, on a selection screen, a plurality of examination selection buttons corresponding to a preset examination pattern, receive a selection operation for any of the examination selection buttons on the selection screen, after receiving the selection operation, transition the display to a second screen on which an observation image is displayed and for adjusting an apparatus state, and execute an examination after passing through display of the second screen, in which identification information indicating a type of the scan pattern is arranged on the examination selection button, and two or more types of the identification information corresponding to two or more scan patterns are arranged on at least one of the examination selection buttons, and in a case where an examination is started as the examination selection button on which two or more types of the identification information are arranged is selected, the controller scans a subject eye with each scan pattern corresponding to the two or more types of the identification information to sequentially acquire OCT data of the subject eye corresponding to each scan pattern.

### BRIEF DESCRIPTION OF DRAWINGS

[FIG. 1] FIG. 1 is a diagram illustrating a schematic configuration of an ophthalmologic imaging apparatus according to an embodiment.
[FIG. 2] FIG. 2 is a diagram illustrating an outline of an optical system of the apparatus according to the embodiment.
[FIG. 3] FIG. 3 is a flowchart illustrating an operation flow of the apparatus according to the embodiment.
[FIG. 4] FIG. 4 is a selection screen according to the embodiment, showing a display example in an OCT/FC mode.
[FIG. 5] FIG. 5 is a selection screen according to the embodiment, showing a display example in an anterior ocular segment OCT mode.
[FIG. 6] FIG. 6 is a selection screen according to the embodiment, showing a display example in the anterior ocular segment OCT mode.
[FIG. 7] FIG. 7 is an alignment screen according to the embodiment, in which a face image is displayed as an observation image.
[FIG. 8A] FIG. 8A is a display example of the face image when detection of a subject eye succeeds.
[FIG. 8B] FIG. 8B is a display example of the face image when detection of a subject eye fails.
[FIG. 9] FIG. 9 is a diagram illustrating a relationship between a region on a face image and a movable range of an imaging unit.
[FIG. 10] FIG. 10 is an alignment screen according to the embodiment, in which an anterior ocular segment image is displayed as an observation image.
[FIG. 11] FIG. 11 is an observation screen according to the embodiment, in which macula multi is selected as a scan pattern.
[FIG. 12] FIG. 12 is a confirmation screen corresponding to FIG. 11.
[FIG. 13] FIG. 13 is an observation screen according to the embodiment, in which macula map is selected as the scan pattern.
[FIG. 14] FIG. 14 is a confirmation screen corresponding to FIG. 13.
[FIG. 15] FIG. 15 is an observation screen according to the embodiment, in which FC imaging is selected.
[FIG. 16] FIG. 16 is a confirmation screen corresponding to FIG. 15.
[FIG. 17] FIG. 17 is a report output screen in the embodiment.
[FIG. 18] FIG. 18 is a setting screen in the embodiment.
[FIG. 19] FIG. 19 is a setting screen in the embodiment.
[FIG. 20] FIG. 20 is a confirmation screen according to a modification, in which a wide-area map (retina map) including macula and optic nerve head is selected as a scan pattern.
[FIG. 21] FIG. 21 illustrates a screen configuration in the related art.

### DESCRIPTION OF EMBODIMENTS

### (Overview)

An embodiment of an ophthalmologic imaging apparatus according to the present disclosure will be described.

The ophthalmologic imaging apparatus according to the embodiment includes at least an imaging unit, a user interface, and a controller (controller in the embodiment). The imaging unit is configured to project and receive light to and from a subject eye. In the following description, for convenience, a unit that includes the imaging unit and is configured integrally with the imaging unit may be referred to as an apparatus main body.

The imaging unit includes all or part of an OCT optical system. For example, at least an objective optical system and an optical scanner of the OCT optical system are disposed in the imaging unit. In the present embodiment, the OCT optical system is configured to scan the tissue of the subject eye with a plurality of scan patterns. The OCT optical system may be configured to irradiate the subject eye with measurement light and detect a spectral interference signal between the measurement light and reference light. The OCT optical system has an optical scanner for scanning the measurement light in a plurality of scan patterns.

The user interface is configured to receive an operation input by an examiner. The user interface may be, for example, a touch panel display attached to the apparatus main body, which may also serve as the display unit. The touch panel display is configured to display a UI element for receiving an operation input and a captured image. However, the present invention is not necessarily limited thereto, and a user interface other than the touch panel display may be adopted.

The controller is a processor configured to control various aspects of the ophthalmologic imaging apparatus (for example, imaging control, display control, and the like). In the present embodiment, the controller is configured to cause the display unit to display various screens, such as a selection screen and a second screen, which will be described later. The controller is configured to control the imaging unit to execute an imaging operation.

The selection screen is displayed to allow the examiner to select an imaging operation. The controller is configured to cause the selection screen to display a plurality of examination selection buttons corresponding to preset examination patterns. The controller is configured to receive a selection operation for any one of the examination selection buttons on the selection screen.

After receiving the selection operation, the display transitions to the second screen. The second screen is a screen on which an observation image of a subject eye is displayed and for adjusting an apparatus state. The controller may be configured to start displaying the observation image at a stage when the display transitions to the second screen. In the present embodiment, the apparatus state may be either an alignment state of the imaging unit with respect to the subject eye or imaging conditions of the OCT optical system. The imaging conditions of the OCT optical system may be, for example, at least one of focus, optical path length difference, polarization, dispersion, and scan density. A UI element for adjusting any one of these apparatus states may be arranged on the second screen. Additionally, the second screen may have UI elements arranged thereon for inputting an operation related to capture.

The observation image may be an observation image of the anterior ocular segment or an observation image of the fundus. The observation image may be a front image of the anterior ocular segment or the fundus. The observation image may also be a real-time OCT image of any of the scan lines included in the selected scan pattern.

The ophthalmologic imaging apparatus may include, for example, an observation optical system for acquiring an observation image of the subject eye. Further, for alignment adjustment, a driving unit for adjusting a positional relationship between the subject eye and the imaging unit may be further provided. While the second screen is displayed, the apparatus state may be manually adjusted according to an operation input or may be automatically adjusted.

The controller is configured to execute the examination after the second screen is displayed. The examination includes at least the capture of OCT data (OCT image).

As described above, in the present embodiment, the selection screen on which the plurality of examination selection buttons are arranged, and the second screen (the observation screen in the example) on which an observation image is displayed and for adjusting the apparatus state are displayed in sequence. Accordingly, this simplifies the procedure for examining any subject by selecting an examination pattern and then adjusting the apparatus state. In addition, since the selection screen is a screen separate from the screen on which the observation image is displayed (for example, the observation screen in the example), the plurality of examination selection buttons on which the identification information is arranged on the selection screen can be arranged so as to occupy a larger space on the screen, and thus the visibility of the identification information can be further improved.

Here, in the present embodiment, identification information indicating the type of the scan pattern is arranged in the examination selection button displayed on the selection screen. Two or more types of identification information corresponding to two or more scan patterns may be arranged on at least one of the examination selection buttons. In a case where an examination is started as an examination selection button on which two or more types of identification information are arranged is selected, the controller scans the subject eye with each scan pattern corresponding to the two or more types of identification information and sequentially acquires OCT data of the subject eye corresponding to each scan pattern. During the examination based on the selected examination pattern, the selection screen may not be displayed and operations may be performed sequentially. As described above, the examiner can easily select an examination pattern that combines a plurality of scan patterns via the examination selection button. The identification information corresponding to each scan pattern is arranged on the examination selection button, and further, for an examination pattern obtained by combining a plurality of scan patterns, the identification information corresponding to each scan pattern is arranged on one single examination selection button, making it easy for the examiner to select the desired examination pattern via the examination selection button. Therefore, the screen configuration of the present embodiment easily improves the efficiency of the examination in the facilities and the like.

In the present embodiment, the identification information indicating the type of the scan pattern may be a graphic symbol. The graphic symbol may be, for example, a graphic representation of a scanning trajectory in each scan pattern. However, the graphic symbol as the identification information is not necessarily limited thereto, and may be any format including a graphic capable of specifying the scan pattern. Additionally or alternatively, one to three words of text may be written as the identification information.

Here, in the apparatus of related art shown in FIG. 21, the UI elements for inputting various operations required from the selection of a scan pattern to the start of capture are arranged on the same screen, and the selected scan pattern can be immediately confirmed by the scan line 535 superimposed on the fundus front image 530.

However, in the present embodiment, the selection screen on which the plurality of examination selection buttons are arranged, and the second screen (the observation screen in the example) on which an observation image is displayed and for adjusting the apparatus state are displayed in sequence. In a case where the examiner erroneously selects a scan pattern on the selection screen, reselection is required, which requires rework. In this regard, in a case where a graphic symbol is used for the identification information, the examiner can more intuitively grasp the content of each scan pattern. That is, when selecting an examination pattern, the fundus front image and the scan pattern superimposed on the image do not necessarily have to be displayed on the selection screen, but the examiner can easily grasp the content of the scan pattern by using the graphic symbols arranged on the examination selection button. As a result, rework due to erroneous selection of the examination pattern is less likely to occur. In addition, it is easier to indicate each scan pattern in a space-saving manner by using the graphic symbol. For this reason, display using graphic symbols is particularly suitable for an apparatus with limited screen size, such as an apparatus with a touch panel display attached to the apparatus main body.

In addition, the ophthalmologic imaging apparatus may include an optical system other than the OCT optical system. For example, a front image capturing optical system may be provided. The front image capturing optical system is an optical system configured to capture a two-dimensional front image of the subject eye. In this case, a pattern including capturing a two-dimensional front image can be selected as the examination pattern. Examples of the examination patterns include a pattern that combines one or more scan patterns by OCT with capturing a two-dimensional front image, and a pattern that only captures a two-dimensional front image. In this case, in addition to the OCT scan pattern, identification information indicating the capture of a two-dimensional front image may be arranged on the examination selection button on the selection screen.

In the present embodiment, after the examination pattern is selected and the screen transitions from the selection screen, the progress status of the imaging operation may be displayed using the identification information. For example, the progress status may be displayed on the confirmation screen. In this case, identification information corresponding to each scan pattern (and imaging operation other than OCT) included in the selected examination pattern may be arranged, and information indicating whether imaging has been completed may be displayed in association with each identification information. In the confirmation screen, the OCT data acquired by any one of the scan patterns corresponding to the selection operation is displayed as an image. Further, a UI element for re-imaging that receives an operation input for instructing re-imaging may be arranged on the confirmation screen. For example, in a case of re-imaging the OCT data, in a case where the UI element for re-imaging is operated, the imaging may be performed using the same scan pattern as the OCT data displayed on the confirmation screen. Further, a UI element for normal termination may be arranged on the confirmation screen. The UI element for normal termination may be operated to start imaging with an uncaptured scan pattern or to complete a series of imaging operations based on the examination pattern in a case where desired OCT data is imaged as a result of confirmation via the confirmation screen. A progress display using the identification information may be displayed on a screen other than the confirmation screen. For example, it may be displayed on the observation screen.

The assignment of the scan pattern for each examination pattern (in other words, the assignment of the scan pattern corresponding to each examination selection button displayed on the selection screen) may be performed via a setting screen. In particular, by making the setting screen a separate screen from the selection screen, operations performed via the selection screen do not become complicated. In this case, on the setting screen that is different from the selection screen, the assignment of the scan pattern corresponding to each examination selection button may be set and changed based on an operation input. The identification information is arranged on each examination selection button according to the assignment of the scan patterns to each examination selection button, which has been set and changed via the selection screen. On the setting screen, identification information may be displayed to indicate each imaging method. Since the identification information indicating each scan pattern is the same between an imaging screen and the setting screen, it is easy to assign the scan pattern.

The ophthalmologic imaging apparatus according to the present embodiment may further include a communication unit (communication unit in the embodiment) for communicating with an external device (for example, a system within a facility). In a case where the controller receives an order for an examination pattern corresponding to one of the examination selection buttons, the examination selection button corresponding to the order may be selected on the selection screen regardless of the selection operation. Accordingly, this allows the examiner to confirm the order and easily start the examination according to the order.

### (Example)

Next, an example of the ophthalmologic imaging apparatus according to the embodiment will be described.

An ophthalmologic imaging apparatus 1 is configured to capture an OCT image of a subject eye E. Further, at least a color fundus image is captured as a two-dimensional reflection image of a fundus Er.

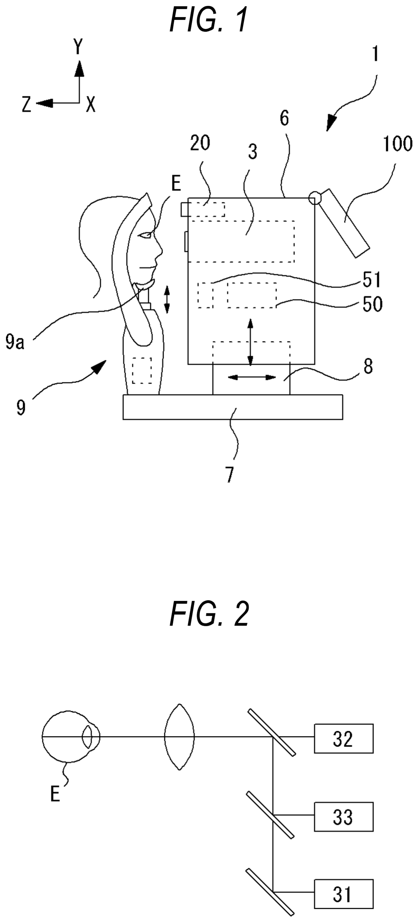

FIG. 1 is an external view of the ophthalmologic imaging apparatus 1. In the example, the ophthalmologic imaging apparatus 1 includes an imaging unit 3, a base 7, a driving unit 8, a face support unit 9, a face imaging camera 20, a controller 50, and a touch panel display (hereinafter, referred to as touch panel) 100. In the following description, for convenience, the unit in which the imaging unit 3 is installed is referred to as an apparatus main body. In the example, the base 7, the driving unit 8, the face support unit 9, the face imaging camera 20, the controller 50, and touch panel 100 are part of the apparatus main body. However, the controller 50 and the touch panel 100 may be units separate from the apparatus main body. The base 7, the driving unit 8, and the face support unit 9 may be omitted from the apparatus main body as appropriate.

In the example, the driving unit 8 is configured to move the imaging unit 3 on the driving unit 8 in each of X, Y and Z directions with respect to the subject eye E. The driving unit 8 includes an actuator for moving the imaging unit 3 in each movable direction, and is driven based on a control signal transmitted from the controller 100. The face support unit 9 is configured to support the face of a subject. The face support unit 9 is fixed to the base 7. The face support unit 9 includes a jaw rest 9a. The jaw rest 9a is movable in an upper-lower direction, thereby adjusting the height of the subject eye E according to the eye level of the apparatus.

The imaging unit 3 mainly includes an optical system illustrated in FIG. 2. As illustrated in FIG. 2, the ophthalmologic imaging apparatus 1 includes an OCT optical system 31, a front imaging optical system 32, and an anterior ocular segment observation optical system 33. As illustrated in FIG. 2, these three optical systems in the example share an objective optical system and are coaxial with each other via a beam splitter/combiner (for example, a half mirror and a dichroic mirror).

The face imaging camera 20 is configured to capture an image of the face of the subject. The controller 100 is configured to identify the position of the subject eye E from the captured face image, and align the imaging unit 3 with the identified position of the subject eye E by controlling the driving of the driving unit 8.

### <OCT Optical System>

The OCT optical system 31 is used to capture an OCT image of the fundus Er. The OCT image may be a two-dimensional tomographic image or a three-dimensional image. Further, as the OCT image, an intensity OCT image may be acquired, or a motion contrast image may be acquired.

In the present example, the OCT optical system 31 is configured to scan the tissue of the subject eye E with the measurement light using a plurality of scan patterns. The OCT optical system 31 may have, for example, two galvanometer mirrors as an optical scanner for scanning with the measurement light. Instead of the galvanometer mirror, for example, a MEMS or an acousto-optical element may also be used.

The OCT optical system 31 has at least a detector, and the detector is configured to detect a spectral interference signal between reference light and return light from the tissue due to the measurement light. The controller 50 is configured to perform predetermined processing on the spectral interference signal to generate an OCT image.

For further details of the configuration of the OCT optical system 31, please refer to, for example, "JP2011-147609A" filed by the present applicant.

### <Front Imaging Optical System>

The front imaging optical system 32 is configured to acquire, as a captured image, a two-dimensional reflection image of the fundus based on the fundus reflection light. The front imaging optical system may be a scanning optical system or a non-scanning optical system. Examples of the scanning optical system include a spot scan type optical system and a line scan type optical system. In the spot scan type optical system, the fundus is two-dimensionally scanned with spot-shaped imaging light. In the line scan type optical system, the scanning is performed with line-shaped imaging light in one direction (details will be described later in a second embodiment). Examples of the non-scanning optical system include an optical system of a general fundus camera. For further detailed configuration of the front imaging optical system 32, please refer to, for example, "JP2019-118721A", "JP2019-141737A", and "JP2008-6105A" filed by the present applicant.

### <Anterior Ocular Segment Observation Optical System>

The anterior ocular segment observation optical system 33 is configured to capture an image of the anterior ocular segment of the subject eye E to acquire an anterior ocular segment observation image. In the present example, the anterior ocular segment observation optical system 33 is configured to illuminate the anterior ocular segment with infrared light as observation light, and a front image of the anterior ocular segment is acquired as the anterior ocular segment observation image based on reflected light. The observation image of the anterior ocular segment is used for alignment and tracking control of the imaging unit 3 with respect to the subject eye E when capturing an image of the fundus.

### <Controller>

The controller 50 is a processing device (processor) configured to perform control processing of each unit and arithmetic processing. The controller 50 includes a CPU, a RAM, a ROM, and the like. For convenience, the controller 50 is configureed to perform image processing on various images acquired by the ophthalmologic imaging apparatus 1. In other words, the controller 50 also serves as an image processing unit.

The controller 50 is electrically connected to each part such as the OCT optical system 31, the driving unit 8, the front imaging optical system 32, the anterior ocular segment observation optical system 33, the face imaging camera 20, the storage unit 51, the touch panel 100, and the like.

The storage unit 51 may be a non-transitory storage medium capable of storing storage contents even when a supply of power is cut off. For example, the storage unit 51 is configured to store various control programs, fixed data, and the like. In addition, for example, the storage unit 51 is configured to store a captured image acquired by the ophthalmologic imaging apparatus 1. The captured image may be transferred to an external storage device (for example, a storage device connected to the controller 50 via a LAN and a WAN).

### <Touch Panel Display>

The touch panel 100 serves as both a monitor and an operation input unit in the ophthalmologic imaging apparatus 1. Various images are displayed on the touch panel 100. In addition, a graphical user interface (GUI) is displayed, and an operation on a UI element (widget) is received from the examiner. Various touch operations are input via the touch panel 100.

### <Detailed Screen Configuration>

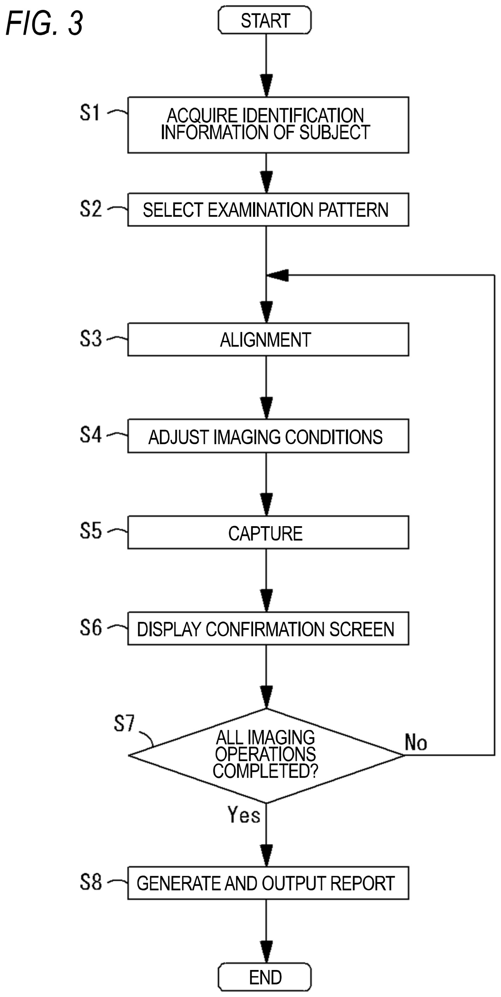

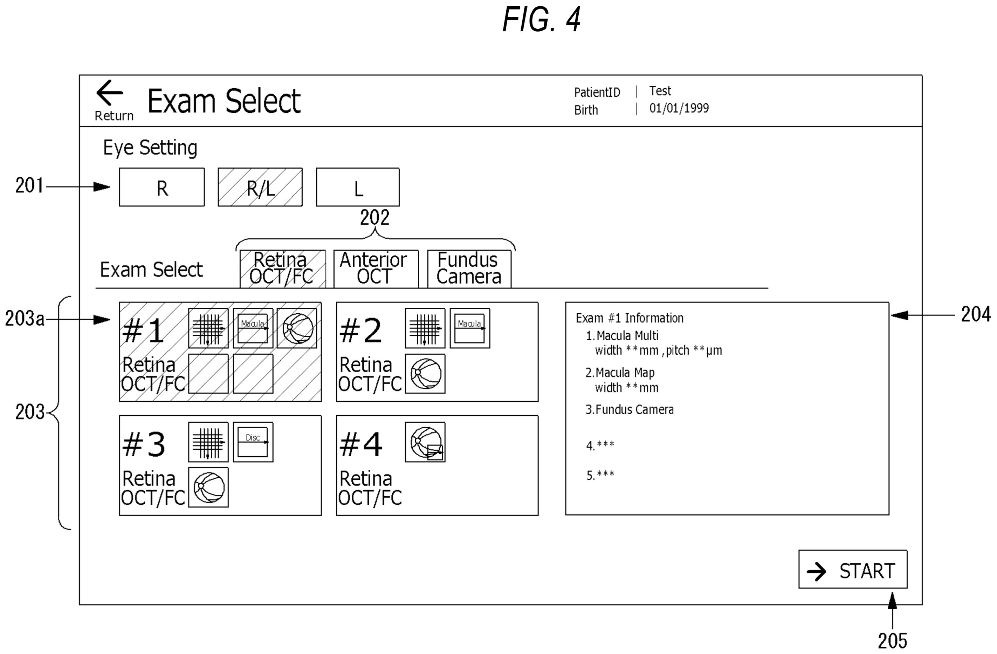

The graphical user interface (GUI) used for imaging in the OCT apparatus 1 will be described along with the flow of operations of the ophthalmologic imaging apparatus 1 in the present embodiment. FIG. 3 is a flowchart showing the flow of operations. FIGS. 4 to 20 illustrate screens displayed on the touch panel 100 at each timing of the operations.

First, identification information (ID, name, age, and the like) of a subject is newly registered or called up (S1). For example, the identification information is input by reading a barcode on which the identification information of the subject is written using a barcode reader connected to the apparatus, or by directly inputting the information via a keyboard. When capturing an image of a subject whose identification information is already registered, the identification information may be called up by displaying a list of subjects and selecting a corresponding subject from the list.

### <Select Examination Pattern>

Next, the controller 50 receives a selection operation for an examination pattern (S2). In this case, an examination selection screen illustrated in FIGS. 4 to 6 is displayed. The content of imaging, including the imaging method, is determined based on the operation input via the examination selection screen.

As illustrated in FIGS. 4 to 6, on the examination selection screen, an eye selection button 201, a mode switching tab 202, an examination selection button 203, and a text box 204 are displayed as UI elements.

The eye selection button 201 is a UI element for selecting the right eye, the left eye, or both eyes as the imaging target. In the example of FIGS. 4 to 6, three buttons are provided, one for the right eye, one for the left eye, and one for both eyes. In a case where the right eye or the left eye is selected as the imaging target, an image of the subject eye E on the selected side is captured using a combo pattern (examination pattern) that is selected separately. In a case where both eyes are selected as the imaging target, an image of one eye is captured using a separately selected combo pattern, and then an image of the other eye is captured using the same combo pattern.

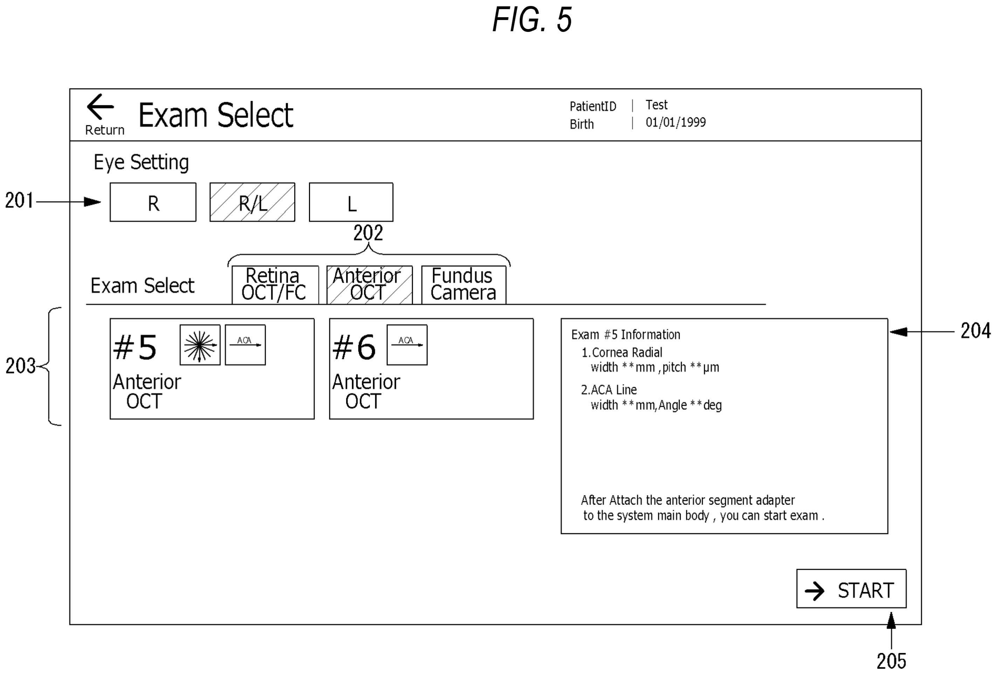

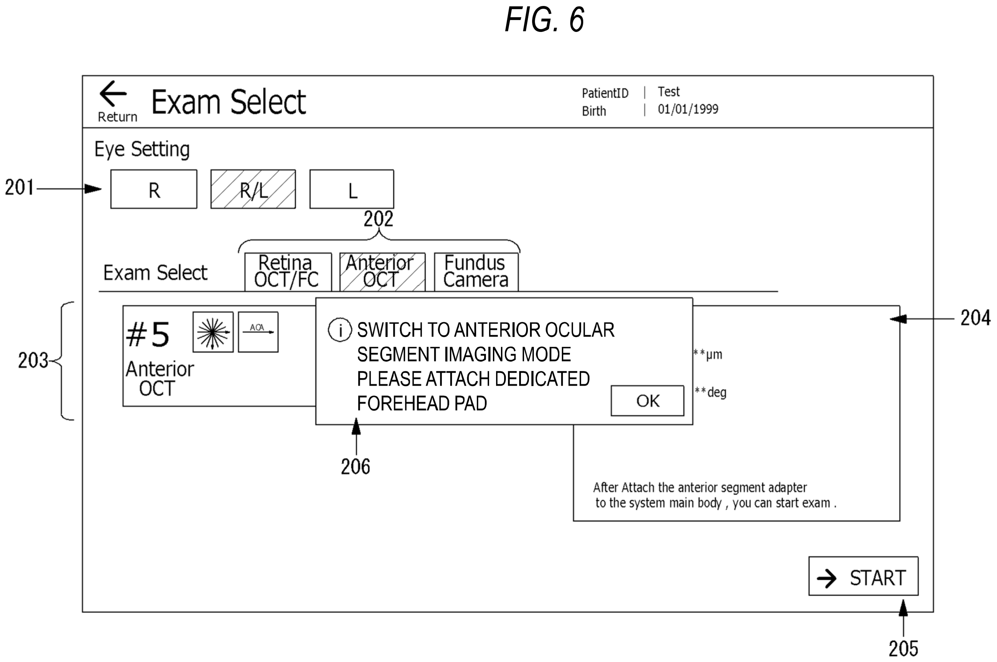

The mode switching tab 202 is operated to switch the imaging mode. In the present example, one of three imaging modes including an OCT/FC mode (see FIG. 4), an anterior ocular segment OCT mode (see FIGS. 5 and 6), and an FC mode (not shown) is selected.

The OCT/FC mode is selected when capturing an OCT image of the fundus.

In the OCT/FC mode, it is also possible to capture a front image of the fundus (either a color fundus image, an infrared image, or an autofluorescence image) in combination with capturing an OCT image.

The anterior ocular segment OCT mode is selected when capturing an OCT image of the anterior ocular segment. The FC mode is selected when capturing a fundus front image without combining OCT imaging.

The examination selection button 203 is a UI element for selecting a combo pattern. Each combo pattern includes one or more imaging operations. A combo pattern including a plurality of imaging operations is prepared for successively performing a plurality of imaging operations on one subject eye E. For example, an example of a combo pattern including a plurality of imaging operations is a pattern that combines a plurality of scan patterns. Each scan pattern specifies a location on the tissue where the OCT image is acquired and the number of OCT images to be acquired. Examples of the type of the scan pattern include line scan, cross scan, multi-scan, radial scan, and raster scan (also referred to as map scan). Further, for each type, a plurality of scan patterns with different positions on the tissue and different scan lengths may be prepared. Further, for example, as an example of a combo pattern including a plurality of imaging operations, there may be a pattern obtained by combining a plurality of imaging operations having different image types. For example, the pattern may be a pattern in which an OCT image and a fundus front image are successively captured, or a pattern in which an intensity OCT image and a motion contrast image are successively captured.

In the present example, a maximum of four buttons are arranged as the examination selection button 203 on the same screen. Each button is associated with one combo pattern. As will be described in detail later, the correspondence between buttons and combo patterns can be set and changed in advance via a setting screen, which will be described later.

As illustrated in FIGS. 4 to 6, identification information corresponding to each imaging operation included in the combo pattern is arranged on each button. In the present example, a graphic symbol is used as the identification information. In the case of a combo pattern including a plurality of imaging operations, a plurality of graphic symbols corresponding to the respective imaging operations are arranged. For example, in a button 203a illustrated in FIG. 4, graphic symbols corresponding to the macula multi and the macula map, which are scan patterns in the OCT imaging, are arranged, and further, a graphic symbol corresponding to capturing of a fundus front image is arranged. For example, as illustrated in FIG. 4, the graphic symbol related to the fundus OCT may indicate a pattern shape (scanning trajectory) of each scan pattern. The examiner can easily grasp a combination of the scan patterns included in the combo pattern via the graphic symbols that indicate a pattern shape of each scan pattern. The graphic symbol corresponding to the capturing of the fundus front image may be a symbol that schematically shows the fundus front image. As described above, in the present example, one or more graphic symbols corresponding to the imaging operations are arranged on each button, thereby indicating the content of the combo pattern corresponding to each button.

In a case where one of the examination selection buttons 203 is selected, the details of the combo pattern corresponding to the selected button are displayed in the text box 204 as text information. That is, the imaging conditions (scanning conditions) for each imaging operation included in the combo pattern are indicated for each imaging operation.

By operating a start button 205 in a state where one of the examination selection buttons 203 is selected, the examination pattern is determined, and the examination selection screen transitions to the next screen (alignment screen in the present example).

In the present example, as illustrated in FIGS. 4 to 6, in a case where the imaging mode is switched based on the operation of the mode switching tab 202, the examination selection button 203 is also changed in conjunction with the imaging mode. For example, as illustrated in FIG. 5, in the anterior ocular segment OCT mode, a button on which a graphic symbol indicating a scan pattern in the anterior ocular segment OCT is arranged is displayed as the examination selection button 203.

In the present example, an anterior ocular segment adaptor (not shown) is attached to the examination window of the imaging unit 3, thereby enabling OCT imaging of the anterior ocular segment. For details of the anterior ocular segment adaptor, please refer to, for example, "JP2011-147609A" filed by the present applicant.

Therefore, in a case where the button corresponding to a combo pattern including the scan pattern of the anterior ocular segment OCT is selected, a message requesting the attachment of the anterior ocular segment adaptor is displayed in the text box 204. In this case, for example, the controller 50 may be configured to detect the attachment or detachment of the anterior ocular segment adaptor in parallel with the display of the examination selection screen. In a case where an anterior ocular segment adaptor is not attached, the controller 50 may disable the operation on the start button 205 even if the selected examination selection button 203 corresponds to the combo pattern that includes the scan pattern of the anterior ocular segment OCT. Further, the message requesting the attachment of the anterior ocular segment adaptor may be output in a case where the operation on the start button 205 is input and the attachment of the anterior ocular segment adaptor is not detected. Further, in the present example, when imaging the anterior ocular segment OCT, a forehead pad for adjusting the working distance is attached to the face support unit 9 together with the anterior ocular segment adaptor. As illustrated in FIG. 6, the detection of the attachment of the anterior ocular segment adaptor may be used as a trigger to further display a message in a pop-up window 206 encouraging the user to attach a forehead pad. Accordingly, it is possible to suitably prevent forgetting to attach the forehead pad when taking an anterior ocular segment OCT image.

### <Alignment>

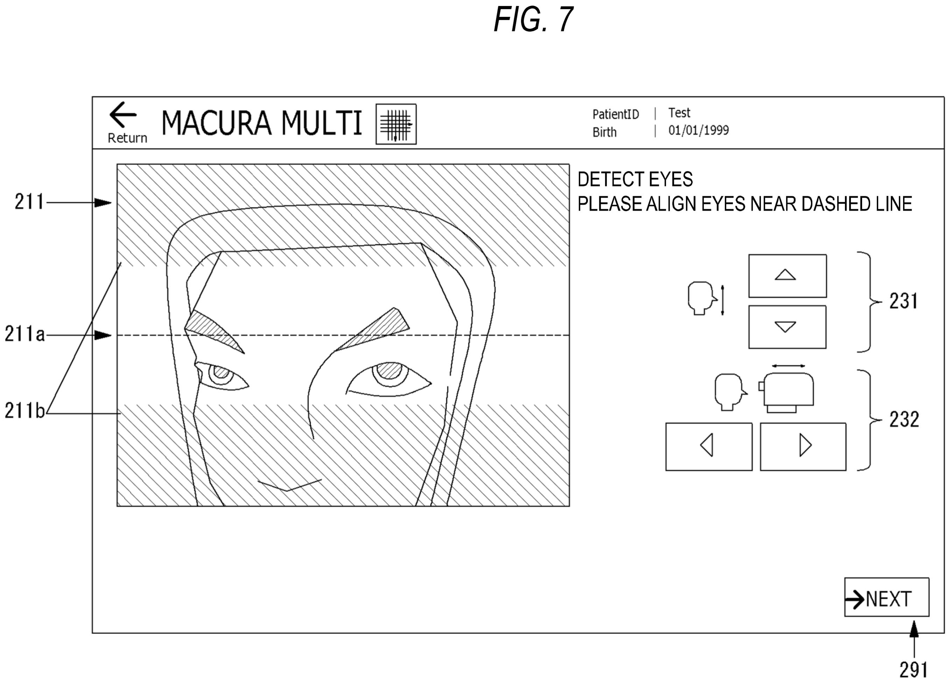

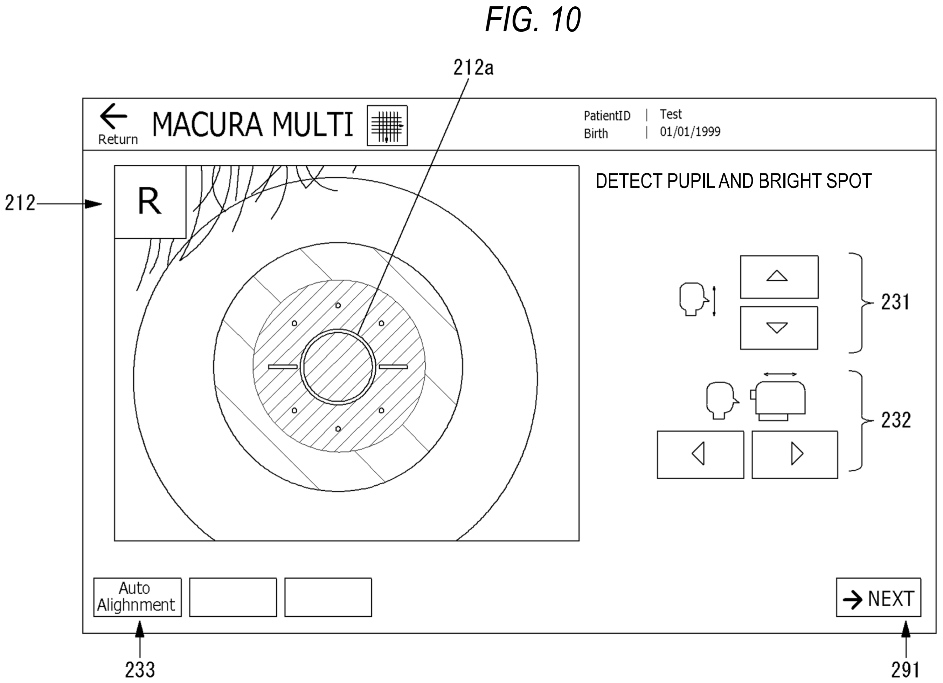

As the examination selection button 203 is selected on the examination selection screen and the start button 205 is operated, the screen transitions to the alignment screen. The alignment screen is shown in FIGS. 7 and 10. In this state, alignment adjustment is performed (S3).

At least an observation image for alignment is displayed on the alignment screen shown in FIGS. 7 and 10. The observation image may be either a face image 211 (see FIG. 7) acquired via the face imaging camera 20 or an anterior ocular segment observation image 212 (see FIG. 10) acquired via the anterior ocular segment observation optical system 33. In parallel with the display of the alignment screen, the controller 50 is configured to adjust the position of the imaging unit 3 relative to the subject eye E based on the observation image to a position where the subject eye E can be captured. In the present example, the position of the imaging unit 3 and the position of the jaw rest 9a in the face support unit 9 are adjusted from a predetermined initial position to a state in which the optical axis of the imaging unit 3 coincides with the subject eye E and the appropriate working distance is obtained.

On the alignment screen illustrated in FIGS. 7 and 10, a jaw rest height adjustment button 231 and a front-rear adjustment button 232 are further displayed. The jaw rest height adjustment button 231 includes a pair of buttons corresponding to the upper and lower directions. As the jaw rest height adjustment button 231 is operated, the controller 50 is configured to move the jaw rest 9a of the face support unit 9 in the upper-lower direction. The front-rear adjustment button 232 is operated to change the position of the imaging unit 3 in a front-rear direction. The front-rear adjustment button 232 includes a pair of buttons corresponding to the front and rear. As the front-rear adjustment button 232 is operated, the controller 50 is configured to move the imaging unit 3 in the front-rear direction.

In the present example, first, as shown in FIG. 7, the face image 211 acquired via the face imaging camera 20 is displayed as an observation image.

The controller 50 is configured to analyze the face image 211 and detect the position of the subject eye E. At this time, a line 211a indicating the reference height of the eye level is displayed at a fixed position in an observation image display region 211. The line 211a is shown as a broken line in FIG. 7. In a case where the position of the subject eye E is far from the line 211a, the examiner operates the jaw rest height adjustment button 231 to bring the subject eye E closer to the line 211a, so that the subject eye E (and the position of the subject eye E) can be detected from the face image 211. The position of the subject eye E may be acquired as two-dimensional coordinates on the face image 211.

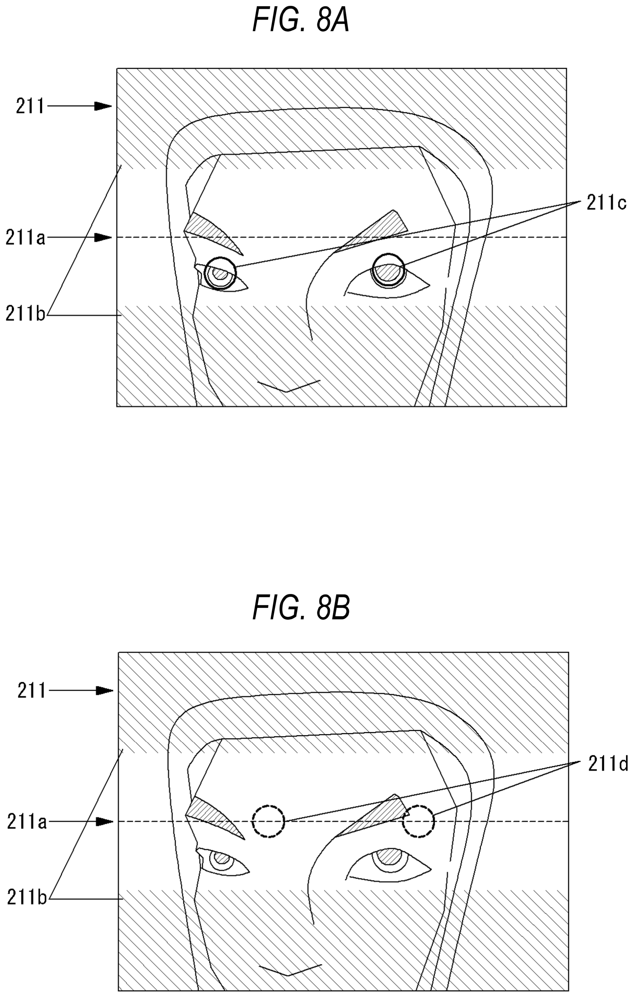

In a case where the subject eye E is detected in the face image 211, a detection result is displayed. In the present example, FIG. 8A is an example of the face image 211 when the detection is successful, and a mark 211c is superimposed on the position of the subject eye E detected in the face image 211. However, due to erroneous detection, the mark 211c may be superimposed on another position such as the eyebrow. In this case, by touching the correct position of the subject eye E, the detection position is corrected to the touched position or to the position of the subject eye E detected in a peripheral region of the touched position. FIG. 8B is an example of the face image 211 when the detection fails. In a case where the detection fails, a mark 211d in a form different from that in the case of success is superimposed at a predetermined position. In FIG. 8B, as an example, the broken line mark 211d is superimposed on the line 211a.

In this case, the detection position can also be corrected by touching the correct position of the subject eye E, as in the above case.

As the position of the subject eye E is detected based on the face image 211, the controller 50 is configured to move the imaging unit 3 from the initial position to execute automatic alignment. For example, the position of the imaging unit 3 is changed in a direction in which the optical axis of the imaging unit 3 (hereinafter referred to as an imaging optical axis) approaches the detected subject eye E. That is, the position of the imaging unit 3 is adjusted, based on the position of the subject eye E detected based on the face image 211.

In the present example, a range in which the imaging unit 3 can move in the upper-lower direction is narrow compared to the angle of view of the face image 211. Even if the subject eye E is detected outside the movable range, the alignment cannot be appropriately performed by moving the imaging unit 3 alone.

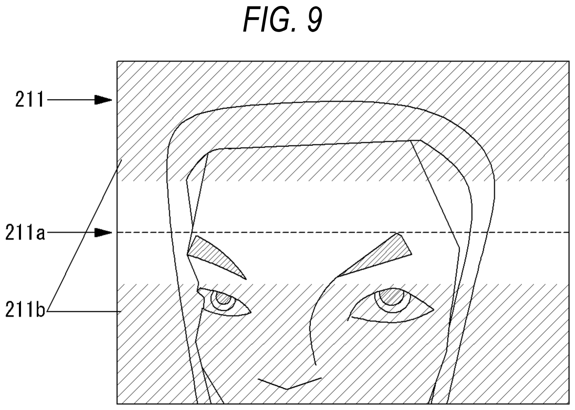

In this regard, in the present example, in a case where the face image 211 is displayed as the observation image, the mask 211b is superimposed on a display region of the observation image. In the present example, the masks 211b are superimposed both above and below the display region. The masks 211b indicate that it is outside the movable range of the imaging unit 3. More specifically, in the present example, a region on which the mask 211b is superimposed is outside the movable range of the imaging unit 3 when the imaging unit 3 is at a predetermined position (for example, the initial position). For example, as illustrated in FIG. 9, when the mask 211b overlaps the subject eye E in the face image 211, the subject eye E is located outside the movable range of the imaging unit 3. In the case of FIG. 9, since the position of the subject eye E is below the movable range of the imaging unit 3, by operating the jaw rest height adjustment button 231 in a direction of raising the jaw rest 9a, the subject eye E can be positioned within the movable range of the imaging unit 3.

As a result, the positional relationship between the imaging unit 3 and the subject eye E can be appropriately adjusted by moving the imaging unit 3. As described above, the mask 211b is superimposed on the region outside the movable range of the imaging unit 3 in the face image 211, so that the examiner can easily grasp whether the height adjustment of the jaw rest 9a is necessary. In a case where the position of the subject eye E on the face image 211 is outside the movable range of the imaging unit 3, the examiner can be appropriately prompted to operate the jaw rest height adjustment button 231.

As described above, in the present example, the mask 211b is superimposed outside the movable range of the imaging unit 3 when the imaging unit 3 is at a predetermined position (for example, the initial position), but the present invention is not necessarily limited thereto, and when the face image 211 is displayed while the imaging unit 3 is moving, the position of the mask 211b on the face image may be changed according to the position of the imaging unit 3.

In the present example, a case has been described in which the examiner determines whether the mask is outside the movable range of the imaging unit 3 by checking the observation image, and adjusts the height of the jaw rest based on the operation of the examiner, but the present invention is not necessarily limited thereto. For example, the controller 50 may be configured to determine whether the position of the subject eye E detected from the face image 211 is outside the movable range of the imaging unit 3, and further, in a case where the position of the subject eye E is outside the movable range of the imaging unit 3, may automatically adjust the height of the jaw rest, or may output guide information via the user interface to prompt the user to adjust the height of the jaw rest 9a. However, when detecting the subject eye E from the face image 211, there may be cases where the subject eye is erroneously detected or the detection fails. In this regard, as in the present example, when the region outside the movable range of the imaging unit 3 is displayed on the observation image in which the face image is displayed, the examiner is prompted to intervene, and the examination may proceed smoothly.

Based on the operation on a transition button 291, the anterior ocular segment observation image 212 is displayed as the observation image (see FIG. 10). In the present example, in a case where an automatic alignment button 233 is selected, the position of the imaging unit 3 is automatically moved with respect to the subject eye E based on the anterior ocular segment observation image 212. In the present example, after an alignment index is projected and the positional relation in each of the XYZ directions is adjusted, the imaging optical axis is adjusted to coincide with the pupil center in the XY direction. In the present example, an indicator 212a is displayed on the anterior ocular segment observation image 212 in a superimposed manner. The form of the indicator 212a changes depending on the alignment state. Thus, the alignment state is shown. In this example, based on a touch operation on the anterior ocular segment observation image 212, the position of the imaging optical axis relative to the subject eye E in the X and Y directions can be adjusted. For example, as a region on the anterior ocular segment observation image 212 in a direction in which the indicator 212a is to be moved is touched, the imaging unit 3 is moved according to the direction. The invention is not necessarily limited thereto, and the imaging unit 3 may be moved so that the center (imaging optical axis) of the indicator 212a is moved to the touched position, or the imaging unit 3 may be moved so that pupil detection is performed with the touched position as the center and the center (imaging optical axis) of the indicator 212a is moved to the center of the detected pupil region.

Hereinafter, unless otherwise specified, the following description will be given by taking as an example a case where the button 203a shown in FIG. 4 is selected in the OCT/FC mode on the examination selection screen.

Therefore, the operation when an OCT image is captured using the macula multi and the macula map, and further a fundus front image is captured will be described.

### <Adjustment of Imaging Conditions>

After the alignment is completed, imaging conditions are adjusted (S4). In the present example, after the alignment is completed, the screen transitions from the alignment screen to the observation screen. In a case where the button 203a shown in FIG. 4 is selected in the OCT/FC mode, an OCT image is first captured using a macula multi scan pattern.

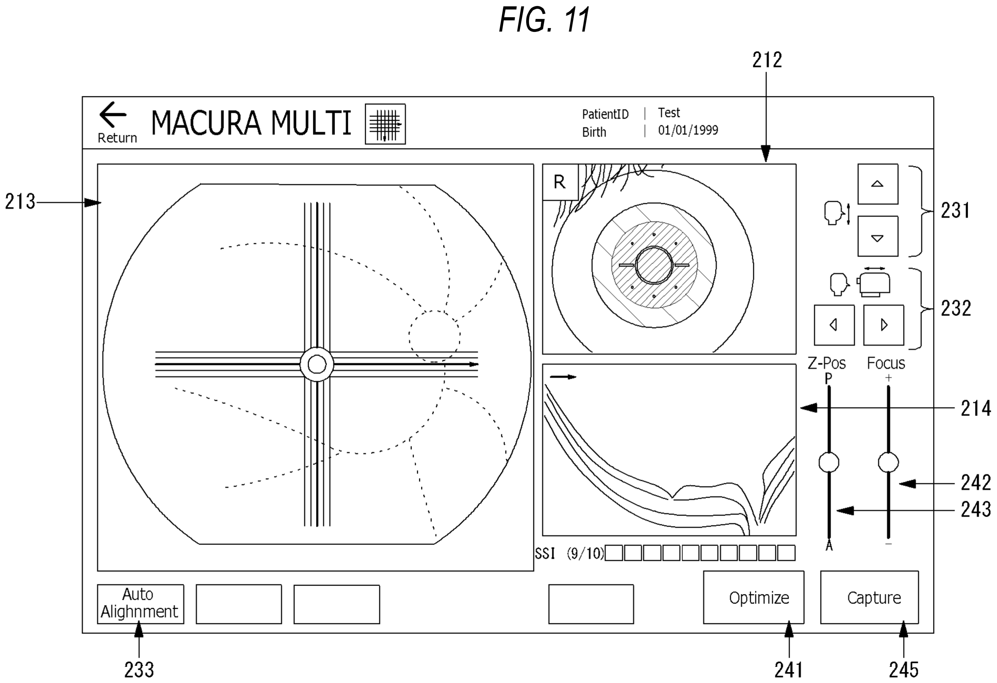

The observation screen displayed in this case is shown in FIG. 11. In the present example, a configuration of the observation screen when capturing the OCT image will be described based on FIG. 11. As illustrated in FIG. 11, in the present example, on the observation screen when capturing an OCT image, a fundus front image 213, a tomographic image 214, a detection condition controller, and a Capture button 245 are displayed on the screen. The fundus front image 213 and the tomographic image 214 displayed on the observation screen before the capture may be observation images acquired in real time. A graph indicating a scan pattern is displayed on the fundus front image 213. With the graph, the examiner can grasp the position where the OCT image is taken.

The imaging condition controller is operated to adjust the detection condition of the OCT optical system 31 and the imaging condition of the front imaging optical system 32. The detection condition is any of various conditions related to the detection ability of ocular tissue, and specific examples include the optical path length difference (hereinafter referred to as OPL), focus, detection sensitivity, polarization state, and the like. Examples of the imaging condition include focus, brightness (any one of light amount, gain, and exposure time), contrast, and the like.

In the present example, an Optimize button 241, a focus controller 242, and an OPL controller 243 are arranged as the imaging condition controller.

In a case where the Optimize button 241 is operated, the controller 50 executes adjustment processing of the OCT optical system 31 and the front imaging optical system 32. In the present example, as an example, each of the OPL, the focus, and the polarization in the OCT optical system 31 and the focus of the front imaging optical system 32 are adjusted to at least a predetermined state. The focus of the OCT optical system 31 and the front imaging optical system 32 may be adjusted based on the fundus observation image (fundus front image 213). In this case, a focus index may be projected onto the fundus, and the focus may be adjusted based on the focus index reflected in the fundus observation image. The results of the adjustment processing are reflected in, for example, the image quality and image position of the fundus front image 213 and the tomographic image 214. In a case where the user checks the fundus front image 213 and the tomographic image 214 and determines that the adjustment has failed, the user can further operate the focus controller 242 and the OPL controller 243 to attempt manual adjustment.

The focus controller 242 and the OPL controller 243 are operated to manually adjust the detection conditions and the imaging conditions, respectively. In the present example, the focus controller 242 and the OPL controller 243 are sliders, and the positions of the "knobs" are moved based on a drag operation or the like. Depending on the position of the knob, the respective imaging conditions and detection conditions are set. For example, the slider of the focus controller 242 and/or the OPL controller 243 is operated according to the depth position of the layer region where it is desired to capture with higher sensitivity.

In addition, in the present example, the anterior ocular segment observation image 212, the jaw rest height adjustment button 231, the front-rear adjustment button 232, and the automatic alignment button 233 are also displayed on the observation screen. Accordingly, the alignment state can be checked even after the transition to the observation screen. If the position of the subject eye E moves from a proper alignment position, the alignment adjustment can be performed automatically or manually.

### <Capture>

The Capture button 245 is configured to accept an operation that serves as a trigger to start capturing. As the Capture button 245 is operated, a scan for imaging is executed with a scan pattern determined in advance (in FIG. 11, a macula multi scan pattern) (S5).

### <Display of Confirmation Screen>

In a case where the imaging is completed, the screen transitions to a confirmation screen. The confirmation screen is a screen for confirming the imaging result. In the present example, an output content of a report may be selected via the confirmation screen.

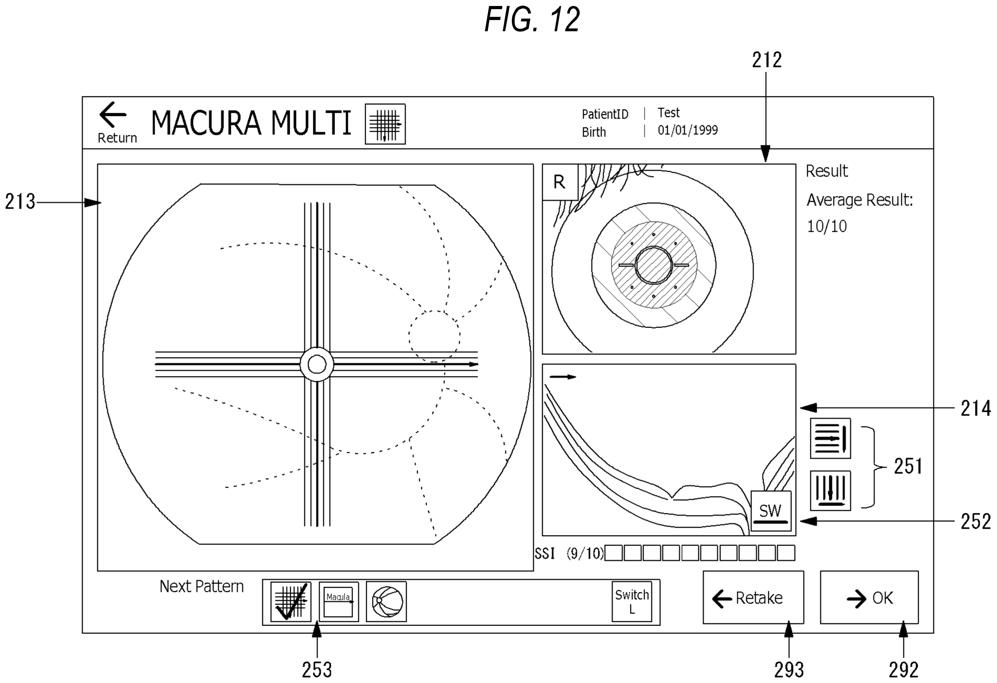

For example, in a case where the imaging by the macula multi scan pattern is completed, the screen illustrated in FIG. 12 is displayed as the confirmation screen. In the present example, in addition to the tomographic image 214 obtained by scanning for imaging, the anterior ocular segment observation image 212 and the fundus front image 213 are displayed on the confirmation screen. The anterior ocular segment observation image 212 and the fundus front image 213 displayed on the confirmation screen may be still images acquired simultaneously with the scanning for imaging. Through the anterior ocular segment observation image 212 and the fundus front image 213, it is possible to confirm the alignment state and the appropriateness of the imaging conditions at the time of imaging.

In a case where the tomographic image 214 is captured for a plurality of scan lines, the tomographic image 214 displayed on the confirmation screen can be switched to an image captured for another scan line. In the present example, a scan line change button 251 is arranged on the confirmation screen. Each time the scan line change button 251 is operated, the tomographic image 214 displayed on the confirmation screen is switched to an image on another scan line in a predetermined order. The tomographic image 214 displayed on the confirmation screen is used as a representative image among the plurality of scan lines. The representative image is an image that is output to a report from among the tomographic images captured for a plurality of scan lines. In the case of the macula multi scan pattern, one representative image is set for each scan line in each of the X and Y directions. The number of representative images and the direction of the scan lines may be determined appropriately for each scan pattern.

By operating a switch button 252, the tomographic image displayed on the screen is switched between the scan line in the X direction and the scan line in the Y direction. Further, by operating a button (not shown), the tomographic image 214 is enlarged and displayed.

The confirmation screen further includes an OK button 292 and a Retake button 293. The retake button 293 is operated to capture a new image. In the present example, in a case where the Retake button 293 is operated, the most recent imaging result is discarded, and the screen transitions to the observation screen. In this case, imaging is performed using the same scan pattern as the most recent imaging.

As the OK button 292 is operated, the imaging result is stored. Further, in a case where the selected combo pattern includes an imaging operation that has not yet been captured, the screen transitions to an observation screen. In this case, the scan pattern or the type of image to be captured is changed to the next one in the order.

The progress (completion status) of the imaging operations included in the selected combo pattern is displayed in a progress display box 253 (progress display region in the example). Specifically, in the progress display box 253, one or more graphic symbols respectively corresponding to the imaging operations included in the combo pattern selected in advance are arranged side by side. The graphic symbols displayed in the progress display box 253 is the same as the graphic symbols displayed in the button selected by the examiner from among the examination selection buttons 203. As shown in FIG. 12, a check mark is superimposed on the graphic symbol corresponding to a completed imaging operation. This allows a distinction to be made between a completed imaging operation and an incomplete imaging operation. Therefore, the examiner can easily grasp the progress status of the imaging operations included in the combo pattern via the graphic symbols.

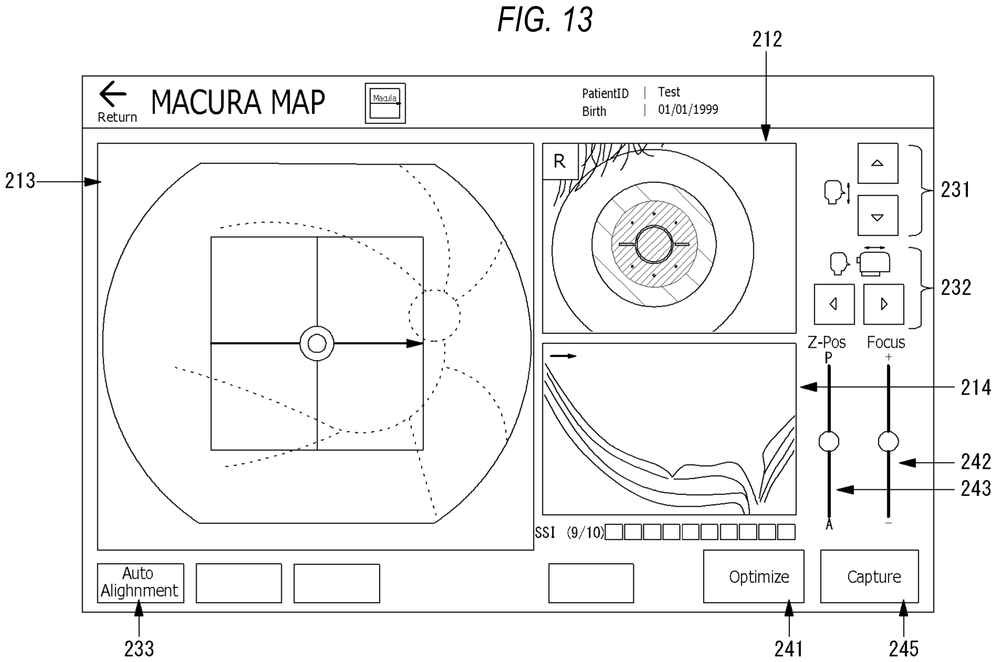

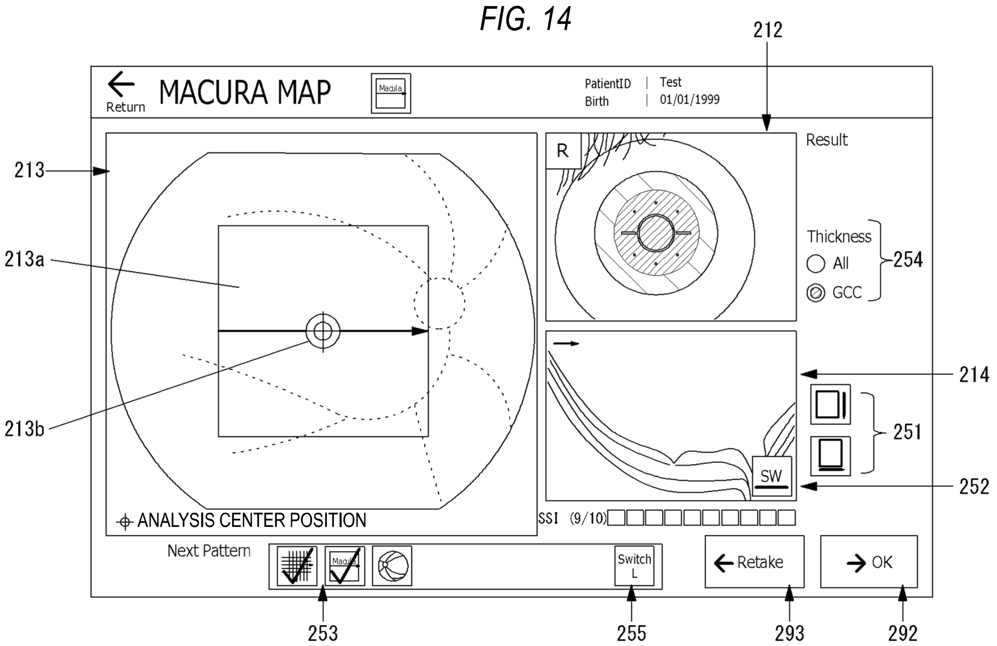

When the OK button 292 is operated on the confirmation screen, in a case where there is an imaging operation that has not yet been executed (S7: No), the process proceeds to the next imaging operation. In a case where the OK button 292 is operated on the confirmation screen in FIG. 12, the process proceeds to the next imaging operation, which is imaging using the macula map. At this time, the controller 50 transitions the screen from the confirmation screen illustrated in FIG. 6 to the observation screen illustrated in FIG. 13. A graph superimposed on the fundus front image 213 represents the scan pattern of the macula map on the observation screen in a case where imaging is performed using the macula map. Other points are the same as those in FIG. 11. As the Capture button 245 is operated, imaging using the macula map is performed. As a result, a three-dimensional tomographic image centered on the macula is captured. In a case where the imaging is completed, the screen transitions to a confirmation screen illustrated in FIG. 14.

In the present example, an operation input for changing and setting an analysis region 213a in the three-dimensional tomographic image can be received via the confirmation screen illustrated in FIG. 14. Specifically, the position of the analysis region 213a can be changed in the X and Y directions via the front image 213. For example, a mark 213b indicating the center position of the analysis region 213a can be moved in response to an operation on the touch panel 100. In the present example, a reference position of analysis processing using a normal eye database is displaced depending on the center position. However, the present invention is not necessarily limited thereto, and the reference position of the analysis processing may be changed. When changing the analysis region 213a in a depth direction, an analysis region change button 254 is operated. As an example, in FIG. 14, as the analysis region change button 254, a button corresponding to the entire retina and a button corresponding to a ganglion cell complex (GCC) are arranged. By selecting one of the buttons, a region corresponding to the selected button is set as the analysis region 213a in the depth direction. The GCC is a composite layer region including a retinal nerve fiber layer (RNFL), a ganglion cell layer (GCL), and an inner plexiform layer (IPL).

The analysis region 213a changed or set via the confirmation screen is reflected in the analysis processing for the imaging result displayed on the confirmation screen. In the present embodiment, as an example, the analysis processing related to a thickness of the tissue is performed. The two-dimensional distribution of measured values of the thickness of the tissue and/or the two-dimensional distribution of the comparison results between the thickness of the tissue of the normal eye and the measured values is output as the result of the analysis processing. For example, the analysis processing may be executed at the timing of generating a report on the imaging results, or may be executed at another timing. The content of the analysis processing is not limited to the analysis processing related to the layer thickness (details will be described later).

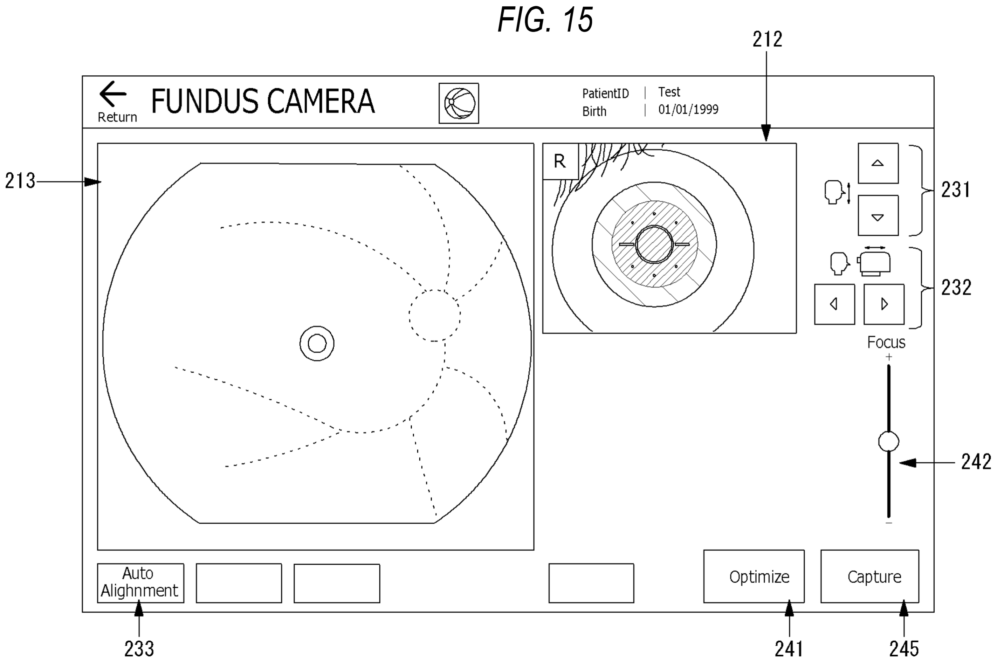

In a case where the OK button 292 is operated on the confirmation screen of FIG. 14, the process proceeds to the next imaging operation, that is, imaging a fundus front image. At this time, the controller 50 transitions the screen from the confirmation screen illustrated in FIG. 14 to the observation screen illustrated in FIG. 15. In the following description, it is assumed that a color image is captured as an example.

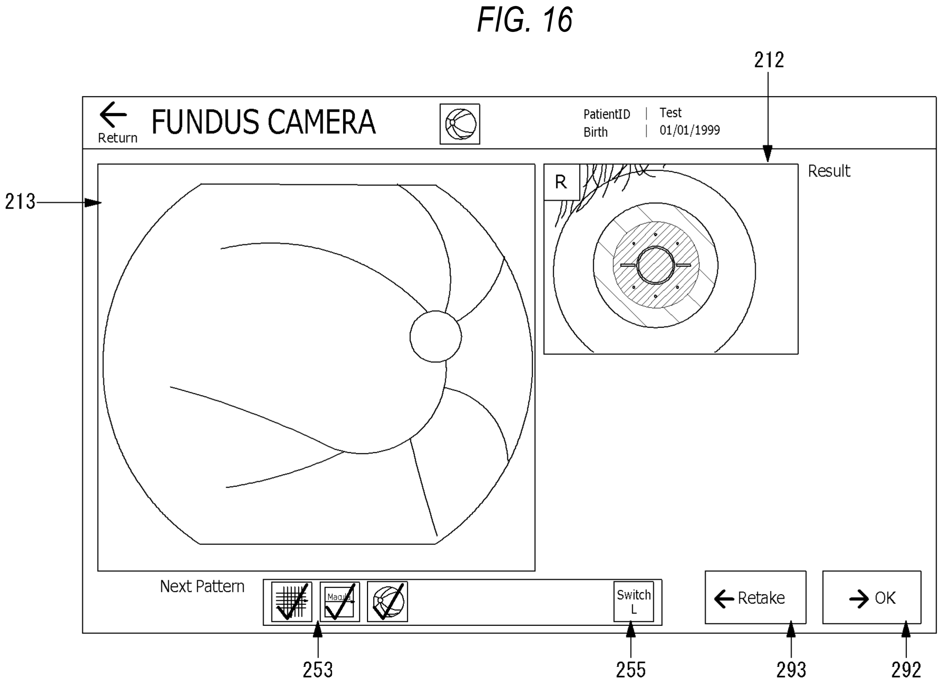

In this case, no further OCT acquisition is performed, and therefore no controllers or display regions related to the OCT are arranged on the observation screen when capturing the fundus front image (color image). Other points are the same as those in FIGS. 11 and 13. When the Capture button 245 is operated, the screen transitions to a confirmation screen shown in FIG. 16.

When the OK button 292 is operated on the confirmation screen illustrated in FIG. 16, in a case where the imaging of the opposite eye has not yet been performed (S7: No), the imaging unit 3 is moved to the opposite eye, and the imaging is repeated for the opposite eye using the same imaging method. In a case where various imaging operations corresponding to the selected examination selection button 203 are completed (S7: Yes), a report output screen is displayed (S8).

In the present example, the graphic symbol displayed in the progress display box 253 of the confirmation screen also serves as a UI element for changing the imaging operation to be performed next. For example, any one of the graphic symbols displayed in the progress display region on the confirmation screen is selected based on an operation input. After the selection, as the OK button 292 is operated, the selected imaging operation is performed. As a result, it is possible to appropriately skip the pattern that does not need to be imaged in the examination or change the order of the examination. In addition, a left-right eye switching button 255 is arranged in the progress display box 253. When the left-right eye switching button 255 is operated, the process proceeds to the imaging operation of the opposite eye. As a result, the left and right eyes can be switched at any stage during the examination.

### <Report Output Screen>

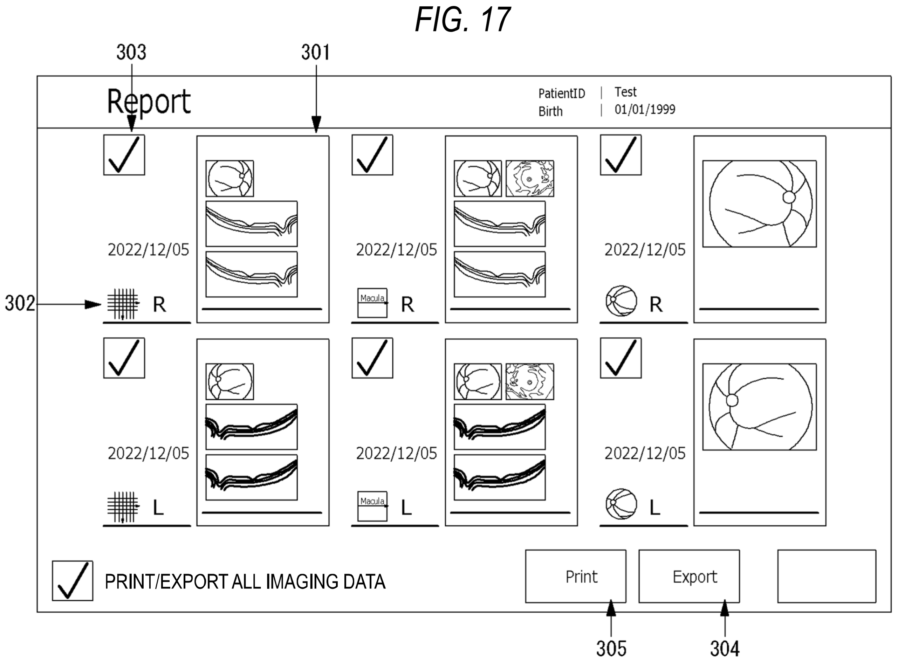

FIG. 17 shows an example of the report output screen. In this example, a report is generated for each imaging operation and each subject eye E. When a macula multi-image, a macula map-image, and a fundus front image are captured for each of the left and right eyes, three types of reports are output for each eye. That is, a total of six reports 301 are generated and displayed on the report output screen.

As shown in FIG. 17, on the report output screen of the present embodiment, a graphic symbol 302 corresponding to the imaging operation on which each report 301 is based is arranged around each report 301. The graphic symbol arranged around the report 301 is the same as the graphic symbol displayed on the button selected by the examiner from among the examination selection buttons 203, so that it is possible to easily grasp, via the graphic symbols, which imaging operation result each report indicates.

On the report output screen, a check box 303 is arranged corresponding to the respective reports 301. By selecting the check box 303, a check mark is added to the report. When any of the reports is added with the check mark, an export button 304 or a print button 305 is operated, and the report with the check mark is output. In addition, by selecting the check box 303 for collective selection, it is possible to add or remove check marks to all reports.

When the export button 304 is operated, the data of the report to which the check mark is added is transferred to a predetermined address. The print button 305 causes a printer (not shown) to print out a report to which the check mark is added.

### <Pre-Set Combo Pattern>

As described above, the combo pattern that can be selected by operating the examination selection button 203 (see FIGS. 4 to 6) can be set and changed in advance.

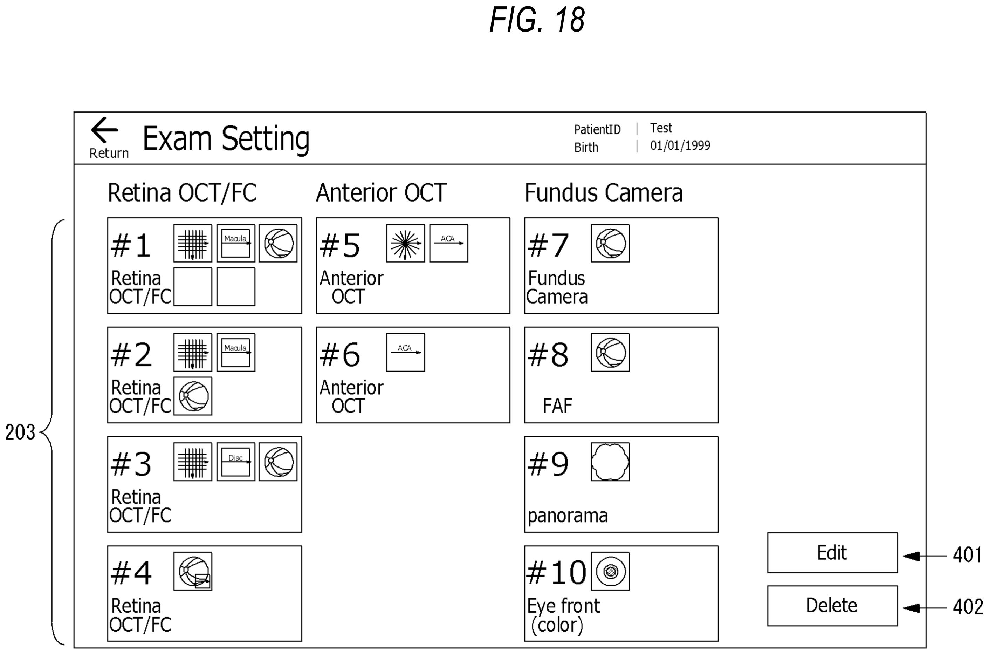

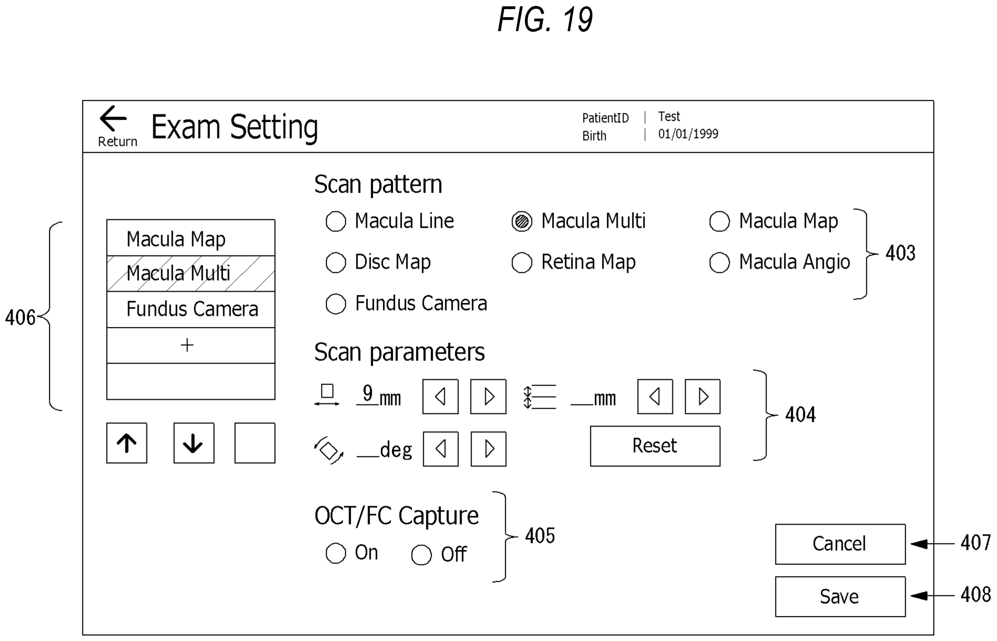

FIGS. 18 and 19 illustrate screens used for setting a combo pattern. The screen illustrated in FIG. 18 displays a list of examination selection buttons 203 that can be selected in each mode.

As any button is selected and an Edit button 401 is operated, the screen transitions to the screen of FIG. 19, and the combo pattern corresponding to the button can be edited. A combo list 406 is displayed on the screen of FIG. 19. By selecting a cell on the combo list 406 that has a "+" displayed and selecting one of the plurality of scan patterns listed in a scan pattern selection section 403, the scan pattern included in the combo list 406 is added. In the case of a scan pattern related to OCT, scan parameters (for example, scan length, scan pitch, angle, and the like) can be changed from default values via a scan parameter setting section 404. In the case of the scan pattern related to OCT, whether to capture a fundus front image in conjunction with the OCT imaging is set via an OCT/FC continuous imaging setting section 405.

In the imaging operation in which On is selected, the fundus front image (color fundus image) is continuously captured after the OCT imaging without requesting a release operation. That is, a continuous pattern of the OCT and the fundus front image is set.

Based on the combo list 406 edited via the screen of FIG. 19, a combo pattern corresponding to each examination selection button 203 is set. In addition, the graphic symbol arranged on each examination selection button 203 is also changed according to the combo pattern.

### <Modifications>

The techniques disclosed in the above embodiment are merely examples. Therefore, it is also possible to change the techniques exemplified in the above embodiment. For example, it is also possible to execute only some of the plurality of techniques exemplified in the above embodiment.

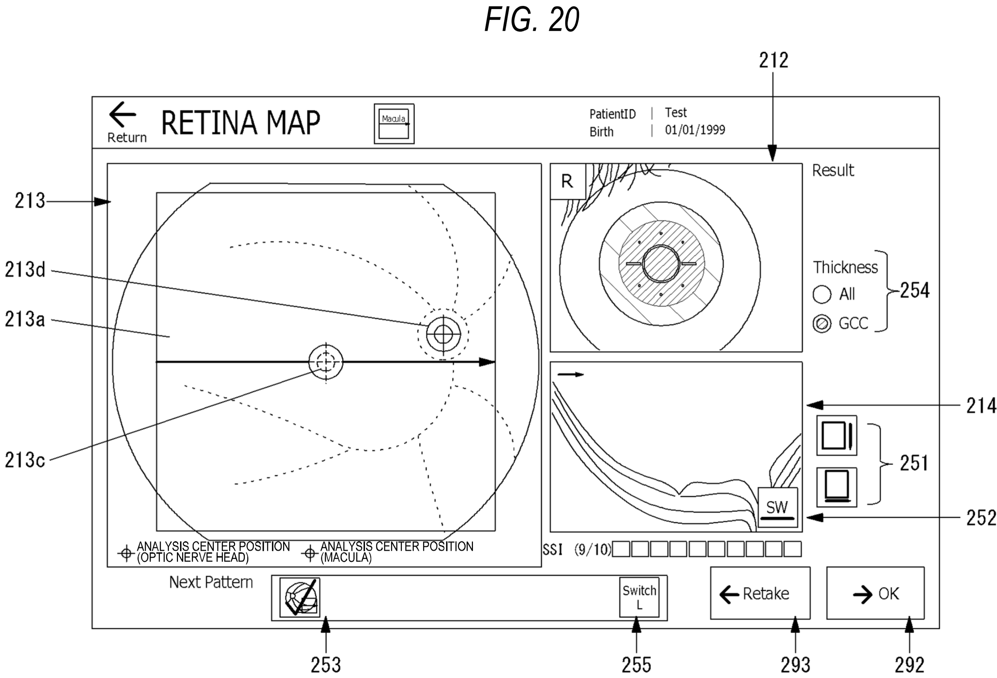

For example, in the above embodiment, in a case where imaging is performed using map scanning, an operation input for changing and setting an analysis region in a three-dimensional tomographic image can be received via the confirmation screen. In the above embodiment, one analysis region centered on the macula is set, but a plurality of analysis regions may be set. For example, in a case where the map scan is performed with a range including the macula and the optic nerve head as the imaging range, the analysis map corresponding to each analysis region may be generated by analyzing the three-dimensional OCT data in each of a first analysis region set around the macula and a second analysis region set around the optic nerve head. In this case, an operation input for changing the reference position for analysis in each of the first analysis region and the second analysis region may be input on the same confirmation screen. For example, as illustrated in FIG. 20, an operation can be input to move a first mark 213c indicating the center position of the first analysis region and a second mark 213d indicating the center position of the second analysis region, so that the reference position of each analysis region can be changed in the X and Y directions. The analysis region in the depth direction may also be separately set between the first analysis region and the second analysis region.

In addition, in the above embodiment, a case where the analysis processing regarding the thickness of the tissue is performed as the analysis processing has been described, but the analysis processing is not necessarily limited thereto, and analysis may be performed on a measurement value (actual measurement value) based on three-dimensional OCT data other than the thickness of the tissue, or a comparison result between the measurement value and the normal eye data may be displayed. The measurement value based on the three-dimensional OCT data may relate to at least one of the thickness, density, shape, and the like of the tissue. For example, the analysis map may be a thickness map of the tissue of the subject eye E (specific examples include a layer thickness map of the fundus, a corneal thickness map, or the like), a density map (specific examples include a blood vessel density map, a cell density map, or the like), a curvature map (topography), a height map (elevation map), or a map showing a two-dimensional distribution of layer thickness information or a two-dimensional distribution of blood vessel information other than those described above. The normal eye data may be statistical data of the normal eye with respect to a measurement value (actual measurement value) based on the three-dimensional OCT data. The comparison result may be information indicating the degree of deviation between the measurement value and the normal eye data, and may be expressed, for example, as a difference, a ratio (for example, a percentile), a deviation value, or the like. The analysis map may also indicate a two-dimensional distribution of changes over time in the measurement values or the comparison results. In the above embodiment, the three-dimensional OCT data that forms the basis of the analysis map indicates the reflection intensity in a three-dimensional region (a two-dimensional region in the XY directions plus the depth direction) of the subject eye E, but is not necessarily limited to this and may also be a three-dimensional motion contrast data.

## Claims

1. An ophthalmologic imaging apparatus comprising:
an imaging unit including all or a part of an OCT optical system configured to scan tissue of a subject eye with a plurality of scan patterns;
a user interface configured to receive an operation input by an examiner; and
a controller configured to:
display, on a selection screen, a plurality of examination selection buttons corresponding to a preset examination pattern;
receive a selection operation for any of the examination selection buttons on the selection screen;
after receiving the selection operation, transition the display to a second screen on which an observation image is displayed and for adjusting an apparatus state; and
execute an examination, after passing through display of the second screen,
wherein identification information indicating a type of the scan pattern is arranged on the examination selection button, and two or more types of the identification information corresponding to two or more scan patterns are arranged on at least one of the examination selection buttons, and
wherein in a case where an examination is started as the examination selection button on which two or more types of the identification information are arranged is selected, the controller scans a subject eye with each scan pattern corresponding to the two or more types of the identification information to sequentially acquire OCT data of the subject eye corresponding to each scan pattern.

2. The ophthalmologic imaging apparatus according to claim 1, wherein the identification information is a graphic symbol.

3. The ophthalmologic imaging apparatus according to claim 1 or 2, further comprising:
an apparatus main body including at least the imaging unit,
wherein the user interface is a touch panel display attached to the apparatus main body, and the user interface also serves as a display unit on which the selection screen is displayed.

4. The ophthalmologic imaging apparatus according to any one of claims 1 to 3,
wherein in a case where a selection operation is received, on the selection screen, for the examination selection button on which two or more types of the identification information are arranged,
after a screen transitions from the selection screen, two or more imaging operations based on scan patterns corresponding to the identification information are sequentially executed without displaying the selection screen in between.

5. The ophthalmologic imaging apparatus according to any one of claims 1 to 4, further comprising:
a communication unit configured to communicate with outside,
wherein in a case where an examination order corresponding to any one of the examination selection buttons is acquired via the communication unit, the examination selection button corresponding to the examination order is selected on the selection screen regardless of the selection operation.

6. The ophthalmologic imaging apparatus according to any one of claims 1 to 5,
wherein after a screen transitions from the selection screen based on the selection operation, a progress status of an imaging operation is displayed using the identification information.

7. The ophthalmologic imaging apparatus according to any one of claims 1 to 6,
wherein after acquiring the OCT data of the subject eye corresponding to each of the two or more types of scan patterns, a list of a plurality of reports generated corresponding to each of the scan patterns is displayed, and identification information corresponding to each report is arranged around each report respectively on a report output screen for receiving a selection operation for a report to be output.

8. The ophthalmologic imaging apparatus according to any one of claims 1 to 7,
wherein on a setting screen different from the selection screen, assignment of the scan patterns corresponding to respective examination selection buttons is set and changed based on an operation input, and
wherein on the selection screen, the identification information is arranged on each examination selection button according to the assignment of the scan patterns to respective examination selection buttons.

9. The ophthalmologic imaging apparatus according to claim 8,
wherein the identification information is displayed on the setting screen to indicate each of the scan patterns.
